(19) Europäisches Patentamt
European Patent Office
Office européen des brevets

(11) **EP 2 630 915 B1**

(12) **EUROPEAN PATENT SPECIFICATION**

(45) Date of publication and mention
of the grant of the patent:
**21.10.2015 Bulletin 2015/43**

(51) Int Cl.:
*A61B 1/00* *(2006.01)*          *A61B 1/06* *(2006.01)*
*A61B 1/313* *(2006.01)*        *A61F 2/00* *(2006.01)*
*A61B 5/107* *(2006.01)*        *A61B 17/00* *(2006.01)*
*A61B 19/00* *(2006.01)*

(21) Application number: **13156689.5**

(22) Date of filing: **26.02.2013**

(54) **Ultra-wide angle zoom projection system for real time in-situ surgical metrology**

Ultraweitwinkel-Zoomprojektionssystem für die Echtzeit-in-situ-OP-Metrologie

Système de projection de zoom à angle ultra-large de métrologie chirurgicale in situ en temps réel

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.02.2012 US 201261603451 P
12.02.2013 US 201313765071**

(43) Date of publication of application:
**28.08.2013 Bulletin 2013/35**

(73) Proprietor: **Covidien LP
Mansfield, MA 02048 (US)**

(72) Inventors:
• **Pinto, Candido Dionisio
  Pacifica, CA 94044 (US)**
• **Stanley, Eric A.
  Milford, CT 06460 (US)**

(74) Representative: **Soames, Candida Jane
Maschio & Soames IP Limited
20 Carlton Crescent
Southampton, SO15 2ET (GB)**

(56) References cited:
EP-A1- 1 480 067          JP-A- 2011 185 767
US-A1- 2008 024 793     US-A1- 2009 270 682
US-A1- 2010 201 796     US-A1- 2011 279 670

EP 2 630 915 B1

## Description

## BACKGROUND

Technical Field

The present disclosure relates to in-situ surgical metrology, and more particularly, to a metrology system including a zoom projection for measuring tissue defect sizes and a method of use therefor.

Background of Related Art

Various surgical procedures are performed in a minimally invasive manner. When compared to the larger openings typically found in traditional procedures, both trauma to the patient and recovery time are reduced for procedures involving small openings. Minimally invasive procedures involves forming a small opening through a body wall of the patient, e.g., in the abdomen, and inserting a seal anchor through the opening to provide a substantially fluid-tight seal between a body cavity of a patient and the atmosphere. Due to the relatively small interior dimensions of the access devices used in endoscopic procedures, only the elongated, small diametered instrumentation may be used to access the internal body cavities and organs. In general, prior to the introduction of the surgical object into the patient's body, insufflation gases are used to enlarge the area surrounding the target surgical site to create a larger, more accessible work area.

Minimally invasive surgery requires a great deal of skill in manipulating the long narrow endoscopic instruments to a remote site under endoscopic visualization. Videoendoscope refers to a flexible or rigid probe enabling the user to see on a video screen the image of a target located in a dark cavity. The measuring methods used in videoendoscopy can be grouped into two categories. Direct measuring methods involve simultaneously displaying on the video screen of the videoendoscopic probe an image of a known size reference associated with the target. Indirect measuring methods involve simultaneously displaying on the video screen of the videoendoscopic probe, the image of the target of unknown size, which is to be measured, and the image of an auxiliary element associated with the target, whose position on the video screen reflects a significant physical parameter of the measurement.

Directly comparing the size of the video image of the unknown element with the size of the video image of the known element is very simple to implement, however, cannot be used universally insofar as it is unusual to have an endoscopic image showing a reference element of known size located next to the target to be measured.

Indirect methods have limitations concerning the depth of the field of measurement, due to the fact that the auxiliary image projected onto the target is clear only at a given observation distance. Beyond this observation distance, the video image of this auxiliary image is substantially blurry, which leads to imprecision in the mapping of the video image, as well as in the measurements derived therefrom. In addition to the issue of varying magnification with image distance, fast degradation due to spherical aberration present on the projection system may quickly derail the efforts to take accurate measurements of the target site.

Accordingly, there is a need for a metrology system used in minimally invasive procedures that enables a surgeon to accurately measure the dimensions of the surgical site of interest to reduce overall surgery time and cognitive burden on the surgeon.

An endoscope for surgical metrology is known from US 2010/201796. General metrology systems in which a pattern is projected on a surface are known from JP 2011-185767, US 2011/279670 and US 2008/024793.

## SUMMARY

In accordance with an embodiment of the present disclosure, there is provided a surgical instrument including an elongate tubular member and a metrology system mounted on the elongate tubular member. In particular, the metrology system includes a mask, a zoom lens assembly and a light element. The mask includes patterns to be projected on a surgical site of interest to measure dimensions thereof. The zoom lens assembly includes a plurality of lens elements, wherein at least one lens element of the plurality of lens elements is movable relative to each other. The light element propagates light beams through the mask and the zoom lens assembly to project the patterns of the mask onto the surgical site of interest.

In an embodiment, the elongate tubular member may include a finger extending distally from a distal end portion of the elongate tubular member. The finger may be configured and adapted to engage a target surgical site. The zoom lens assembly may have an optical magnification power of at least ten. Furthermore, the zoom lens assembly may have an optical magnification power up to one hundred. The light element may be a collimated emitter. In addition, the light element may include a laser diode. The light element may produce a light beam parallel to a longitudinal axis defined by the surgical instrument. Alternatively, the light element may produce a divergent light beam. In another embodiment, the patterns of the mask may be semi-transparent. In particular, the patterns of the mask may be a series of uniformly spaced concentric circles.

The handle member may include a dial operably coupled with the zoom lens assembly, wherein rotation of the dial results in change in magnification of the mask patterns projected on the surgical site of interest. The handle member may further include a LCD display for viewing the target surgical site and the mask patterns projected thereon. The handle member may include a power supply for the light element. In an embodiment, the surgical instrument may be an endoscope. In another embodiment, the target surgical site and the mask patterns projected thereon may be remotely displayed on an external terminal including a LCD display.

In accordance with another aspect of the present disclosure, there is provided a method of measuring dimensions of a target surgical site. The method includes providing a surgical instrument including a metrology system having a mask, a zoom lens assembly and a light element. In particular, the mask includes patterns to be projected on the target surgical site to measure dimensions of the target surgical site. The zoom lens assembly includes a plurality of lens elements, wherein at least one of the lens elements of the plurality of lens elements is movable relative to each other. The light element propagates light beams through the mask and the zoom lens assembly to project the patterns of the mask onto the target surgical site. The method further includes introducing the metrology system into a body cavity, emitting the light beam through the mask and the zoom lens assembly to project the patterns of the mask onto the target surgical site, and measuring the dimensions of the target surgical site.

In an embodiment, measuring the dimensions of the target surgical site may include adjusting the magnification of the patterns of the mask projected on the target surgical site. In particular, adjusting the magnification of the patterns of the mask projected on the target surgical site may include circumscribing the target surgical site within the projected patterns of the mask. In addition, introducing the metrology system into a body cavity may include inserting the metrology system into the body cavity through a seal anchor in a sealing relation with an opening in tissue.

The method may further include repairing tissue defect after measuring the dimensions of the target surgical site. In particular, repairing tissue defect may include placing a surgical mesh to tissue defect. In addition, repairing tissue defect may include selecting an appropriately sized mesh based on the measured dimensions of tissue defect. Placing a surgical mesh to tissue defect may include aligning the surgical mesh with the patterns projected on tissue defect.

The method may further include sending dimensions of tissue defect to a rapid prototype weaving machine for instantaneously customizing mesh for a patient. In addition, the method may further include sending measurement data to hand-held devices. The method may further include insufflating the body cavity.

In an embodiment, the surgical instrument may further include a dial operatively configured and adapted to adjust magnification power of the zoom lens assembly. The dial may include indicia indicating scale of magnification. In addition, the surgical instrument may further include an LCD display for viewing the target surgical site and the mask patterns projected thereon. In another embodiment, the target surgical site and the mask patterns projected thereon may be remotely displayed on an external terminal including a LCD display. The zoom lens assembly may have an optical magnification power of at least ten. The zoom lens assembly may have an optical magnification power up to one hundred. The surgical instrument may be an endoscope.

## BRIEF DESCRIPTION OF THE DRAWINGS

Various embodiments of the present disclosure are described hereinbelow with reference to the drawings, wherein:

FIG. 1 is a perspective view of a surgical instrument including a metrology system in accordance with an embodiment of the present disclosure;
FIG. 2 is schematic view of the metrology system of FIG. 1;
FIG. 3 is a side cross-sectional view of the surgical instrument of FIG. 1 illustrating use of the metrology system in a body cavity of a patient;
FIG. 4 is a top, plan view of tissue defect illustrating patterns of a mask of the metrology system of FIG. 2 projected thereon; and
FIG. 5 is a schematic view of the surgical instrument of FIG. 1 in use with various remote terminals.

## DETAILED DESCRIPTION OF EMBODIMENTS

Embodiments of the present disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the present disclosure in unnecessary detail.

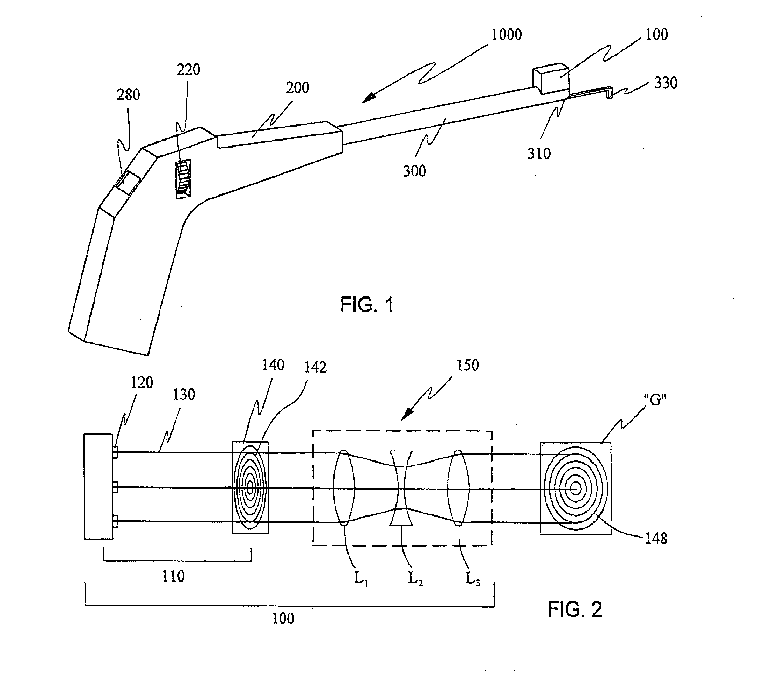

With reference to FIG. 1, there is illustrated a surgical instrument 1000 including a metrology system 100 in accordance with an embodiment of the present disclosure. Metrology system 100 is utilized to measure defect sizes in tissue and to correlate such measurement with, e.g., commercially available mesh sizes during ventral hernia repair, by projecting translucid or semi-translucid pattern/scale directly onto the defect in tissue. Surgical instrument 1000 includes a handle member 200 and an elongate tubular member 300 extending distally therefrom. In particular, metrology system 100 is removably mounted on a distal end portion 310 of elongate tubular member 300. Moreover, distal end portion 310 of elongate tubular member 300 includes a finger 330 extending distally from distal end portion 310. Finger 330 is configured and adapted to enable positioning of metrology system 100 at a predetermined distance away from the surgical site of interest, as will be discussed hereinbelow.

With reference now to FIG. 2, metrology system 100 includes a projector assembly 110 and a zoom lens assembly 150. Projector assembly 110 includes light emitters 120 such as, for example, LED, laser diode or any combination thereof, and a mask 140. Each light emitter 120 emits a light beam 130 for creating a light pattern on a target site "G." Adjacent light beams 130 have a fixed distance therebetween. Light beams 130 may be collimated for increased precision of light pattern. Light beam 130 may be any suitable form of light, such as coherent, partially coherent, visible, infrared, or ultraviolet. Light beam 130 has a wavelength of, for example, 532 nm, to differentiate light beams 130 from a color of any naturally occurring tissue in the human body. Light emitters 120 are powered by a power source (not shown) disposed in handle member 200. The power source may be a standard commercial battery pack.

Mask 140 is semi-transparent and has a substantially opaque mask pattern 142 thereon. Mask patterns 142 have markings of known distances therebetween. For example, mask pattern 142 may be a series of uniformly spaced concentric circles.

Projection of translucid object onto a surgical target surface at a known distance is governed by the following simplified form of the Lens Law:

$$1/S_2 = 1/S_1 + 1/f \qquad (1)$$

wherein, $S_2$ is the distance from the lens to the image plane, $S_1$ is the distance from the translucid object or pattern to be projected on the image plane to the lens, and f is the focal length of the given lens. In addition, magnification of the image with respect to the object is given by the relation:

$$M = S_2 / S_1 \qquad (2)$$

wherein, M is the magnification factor. The image size is related to the object size, and is given by the relation:

$$Y_{image} = Y_{object} \times M \qquad (3)$$

wherein, $Y_{image}$ is the size of the projected image, $Y_{object}$ is the size of the translucid object, and M is the magnification factor.

According to Eqs. (1), (2), and (3), as one moves a given image plane away from its ideal mapping plane, the image will both go out of focus and change sizes very quickly, due to fast variations of the $S_2$ parameter. However, by properly changing the focal length f of the assembly, (e.g., by utilizing a zoom lens assembly), the image plane distance $S_2$ can be kept constant, while enabling change in magnification in a controlled manner.

With continued reference to FIG. 2, metrology system 100 includes zoom lens assembly 150. Zoom lens assembly 150 illustrates a simple 3-element zoom lens. However, it is contemplated that more lens elements may be used for more practical design implementations. Zoom lens assembly 150 includes three lens elements $L_1, L_2, L_3$. Zoom lens assembly 150 is operatively coupled with a dial 220 rotatably disposed in handle member 200. In particular, rotation of dial 220 moves at least one of lens elements $L_1, L_2$ to or from lens element $L_3$, whereby variation of the magnification of mask patterns 142 is effected on target surgical site "G."

Zoom lens assembly 150 has a minimum magnifying power of ten (10X) and up to one hundred (100X) with respect to the original mask pattern 142. For example, when zoom lens assembly 150 is in the smallest magnification state (10X), projection of mask 140 having a diameter of, for example, about 3.0 mm, to target surgical site "G" about 10.0 cm away results in a projected mask pattern 148 having a diameter of about 3.0 cm (10X the size of the original mask pattern 142). At the instrument's smallest magnification state, the depth of focus of the projection system is not smaller than about 5.0 mm and is not larger than about 1.0 cm.

The surgeon could then turn dial 220 to "zoom" the projected mask pattern 148 to a magnification level where target surgical site "G" is circumscribed by projected mask pattern 148, while distance $S_2$ remains constant. With distance parameter $S_2$ optimized and fixed, the desired magnification is affected based on the best focus achieved by system 100 at its smallest magnification state. For example, mask pattern 142 having a diameter of about 3.0 mm can be projected on target surgical site "G" at a maximum magnifying power of one hundred (100X), while having a fixed distance parameter of $S_2$, resulting in a projected mask pattern 148 having a diameter of about 30.0 cm. At maximum magnifying power, the zoom angle is larger than about 56.3 degrees.

Dial 220 includes indicia (not shown) indicating scale of magnification such that the scale of magnification with respect to the original mask pattern 142 can be directly read from dial 220, and thereby enabling the surgeon to readily ascertain the dimensions of the surgical site of interest.

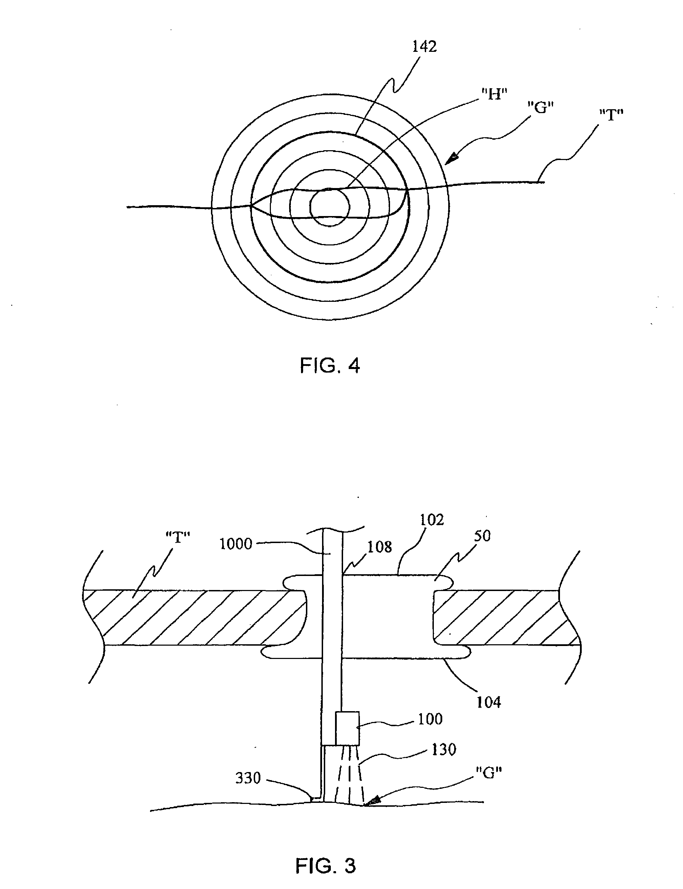

For example, in a ventral hernia repair, the surgeon can turn dial 220 to vary magnification of projected mask pattern 142 to circumscribe the site of ventral hernia "H" therein, as shown in FIG. 4. Based on the indicia provided on dial 220, the surgeon can readily obtain the scale of magnification with respect to the original projection pattern 142, which in turn enables the surgeon to ascertain the dimensions of ventral hernia "H."

With brief reference back to FIG. 1, elongate tubular member 300 includes finger 330 distally extending from a distal end portion 310 of tubular member 300. When metrology system 100 is introduced into the body cavity of the patient, the surgeon positions surgical instrument 1000 such that finger 330 engages target surgical site "G." In this manner, distance $S_2$ from lens $L_3$ of zoom lens assembly 150 to the image plane remains constant, whereby magnification of mask pattern 142 can be effected on target surgical site "G" in a controlled manner. Stated differently, the image plane is set to be positioned at a distal end of finger 330, which engages target surgical site "G." It is also envisioned that the length of finger 330 may be adjustable by the surgeon based on the surgical procedure being performed.

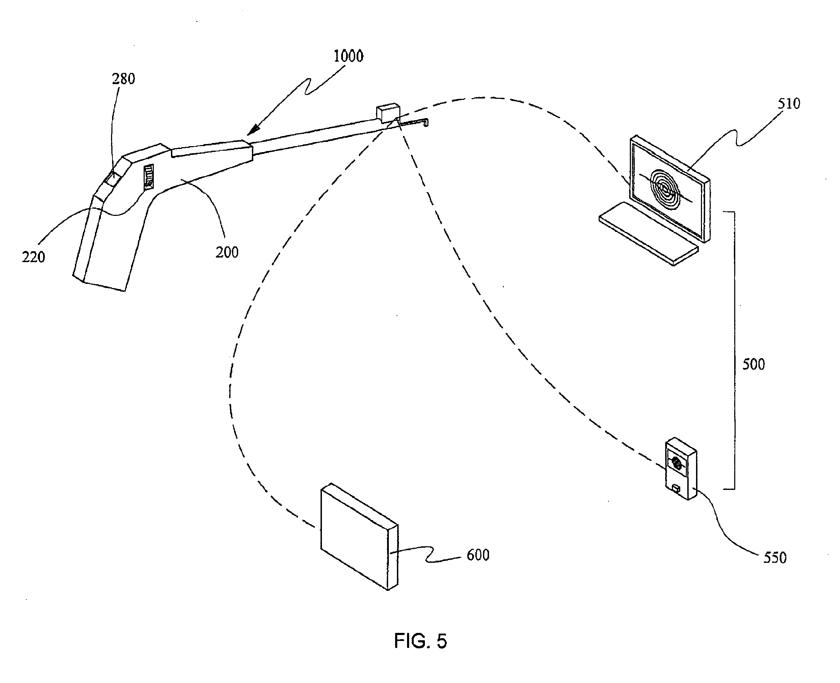

With reference to FIG. 5, the video output of target surgical site "G" and the projected mask patterns 148 projected on target surgical site "G" may be sent to an external terminal 500 including a LCD display 510 and a hand-held device 550. However, the output may also be sent to a LED-lit LCD display 280 mounted on handle member 200. Metrology system 100 may be wirelessly connected with display 280 and external terminal 500 via a standard communications protocol such as, e.g., Bluetooth®. Metrology system 100 can send the dimensions of the tissue defect to an on-site or a remotely located sterile laser cutter or rapid prototype weaving machine 600 that would instantaneously create a customized mesh for the patient. Optionally, optimization algorithms may be utilized to biomechanically optimize tack and suture geometry for the best possible match between meshes and hernia defects. Such feature may be applied in open or laparoscopic procedures, and may utilize multiple references on the mesh including, for example, centroids, cardinal points, optimal annuli and linear fixation distribution patterns.

In applications where direct imaging of a project scale is desired, a landscape meniscus may be directly coupled to a small camera sensor, wherein the sensor can function as an aperture stop and field stop to the system itself.

In use, distal end portion 310 of surgical instrument 1000 is inserted into a body cavity through an opening in tissue "T" until finger 330 engages target surgical site "G," as shown in FIG. 3. In this manner, the distance between finger 330 and metrology system 100 is fixed and remains constant. Alternatively, a seal anchor 50 (FIG. 3) may be utilized to stably position surgical instrument 1000 in the body cavity of the patient. Seal anchor 50 is configured to receive surgical instruments of varying diameter through at least one lumen or channel 108 that extends longitudinally between proximal and distal end portions 102, 104 of seal anchor 50. Seal anchor 50 is formed from elastic/compressible type material having sufficient compliance to form a seal about a surgical object and to establish a sealing relation with the opening in tissue "T." Furthermore, such material enables seal anchor 50 to accommodate off-axis motion of the surgical object extending therethrough.

Surgical instrument 1000 may be inserted through lumen 108 defined in seal anchor 50 positioned within the opening in tissue "T." Lumen 108 compresses against elongate tubular member 300 in a sealing relation therewith. In addition, seal anchor 50 forms a fluid-tight seal against tissue " "T."

Upon proper placement of surgical instrument 1000 in the body cavity of the patient, light emitters 120 emit light beams 130 to create projected mask pattern 148 on target surgical site "G" through zoom lens assembly 150. Initially, zoom lens assembly 150 is set at a minimum magnifying power of ten (10X), wherein mask pattern 142 having a diameter of 3.0 mm is projected onto target surgical site "G," resulting in a projected mask pattern 148 having 3.0 cm diameter. At this time, dial 220 on handle member 200 can be turned to increase magnifying power to enlarge projected mask pattern 148 until the defect in tissue such as, e.g., herniated portion of tissue "T," is enclosed by the enlarged mask pattern 148 projected thereon. With distance parameter $S_2$ optimized and fixed, the desired magnification can be affected based on the best focus achieved by system 100 at its smallest magnification state.

Dial 220 may include indicia indicating scale of magnification such that the scale of magnification with respect to the original mask pattern 142 can be directly read from dial 220 to thereby enable the surgeon to readily ascertain the size of the given surgical site (or tissue defect). In addition, the herniated region and mask pattern projected 148 thereon may be viewed on LCD display 280 mounted on handle member 200.

At this time, the surgeon may perform repair of the herniated region in tissue. For example, mask pattern 148 projected on the herniated region can serve as a guide for correct mesh placement. Moreover, the measurement taken by metrology system 100 can be sent to a sterile laser cutter or rapid prototype weaving machine 600 that would instantaneously customize mesh solution for the patient on site. Such mesh may be used to repair the herniated region in tissue.

Although the illustrative embodiments of the present disclosure have been described herein with reference to the accompanying drawings, the above description, disclosure, and figures should not be construed as limiting, but merely as exemplifications of particular embodiments. For example, in applications where triangulation and/or distance sensing is desirable, a fringe-counting heterodyne interferometer may be utilized with the aid of a LED or laser source, along with a simple Si or GaAs-based sensor. It is to be understood, therefore, that the disclosure is not limited to those precise embodiments, and that various other changes and modifications may be effected therein by one skilled in the art without departing from the scope of the disclosure.

**Claims**

1. A surgical instrument for use in minimally invasive surgery, comprising:

a handle member (200) and

an elongate tubular member (300) extending distally therefrom,

a metrology system (100) mounted on the elongate tubular member (300) at a distal end portion thereof,

**characterised in that** the metrology system (100) comprises

a mask (140) including patterns (142) to be projected on a surgical site of interest to measure dimensions thereof;

a zoom lens assembly (150) including a plurality of lens elements, at least one lens element of the plurality of lens elements being movable;

a light element (120) for propagating light beams through the mask (140) and the zoom lens assembly (150) to project the patterns (142) of the mask (140) onto the surgical site of interest, and wherein the handle member (200) includes a dial (220) operably coupled with the zoom lens assembly (150), and wherein rotation of the dial (220) results in change in magnification of the mask (140) patterns (142) projected on the surgical site of interest.

2. The surgical instrument according to claim 1, wherein the elongate tubular member (300) includes a finger (330) extending distally from a distal end portion of the elongate tubular member (300), the finger (330) configured and adapted to engage a target surgical site.

3. The surgical instrument according to claim 1 or claim 2, wherein the zoom lens assembly (150) has an optical magnification power of at least ten.

4. The surgical instrument according to any preceding claim, wherein the light element (120) is a collimated emitter.

5. The surgical instrument according to any preceding claim, the patterns (142) of the mask (140) are semi-transparent.

6. The surgical instrument according to any preceding claim, wherein the patterns (142) of the mask (140) are a series of uniformly spaced concentric circles.

7. The surgical instrument according to any preceding claim, wherein the handle member (200) further includes a LCD display (280) for viewing the target surgical site and the mask (140) patterns (142) projected thereon.

8. The surgical instrument according to any preceding claim, wherein the light element (120) produces a light beam parallel to a longitudinal axis defined by the surgical instrument.

9. The surgical instrument according to any preceding claim, wherein the light element (120) produces a divergent light beam.

10. The surgical instrument according to any preceding claim, wherein the surgical instrument is an endoscope.

## Patentansprüche

1. Ein chirurgisches Instrument zur Verwendung bei einer minimalinvasiven Chirurgie, aufweisend:

ein Handgriffteil (200) und

ein längliches, röhrenförmiges Teil (300), das sich distal davon erstreckt,

ein Metrologiesystem (100), das an dem länglichen, röhrenförmigen Teil (300) an einem distalen Endabschnitt davon befestigt ist,

**dadurch gekennzeichnet, dass** das Metrologiesystem (100) aufweist:

eine Maske (140), die Muster (142) beinhaltet, die auf eine chirurgisch interessante Stelle projiziert werden sollen, um Abmessungen davon zu messen;

eine Zoomlinsenanordnung (150), die eine Vielzahl von Linsenelementen beinhaltet, wobei zumindest ein Linsenelement von der Vielzahl von Linsenelementen beweglich ist;

ein Lichtelement (120) zur Ausbreitung von Lichtstrahlen durch die Maske (140) und die Zoomlinsenanordnung (150), um die Muster (142) von der Maske (140) auf die chirurgisch interessante Stelle zu projizieren, und

wobei das Handgriffteil (200) ein Einstellrad (220) beinhaltet, das betriebsmäßig mit der Zoomlinsenanordnung (150) verbunden ist, und wobei eine Drehung von dem Einstellrad (220) zu einer Veränderung in der Vergrößerung von den Masken-(140)-Mustern (142), die auf die chirurgisch interessante Stelle projiziert werden, führt.

2. Das chirurgische Instrument gemäß Anspruch 1, wobei das längliche, röhrenförmige Teil (300) einen Finger (330) beinhaltet, der sich distal von einem distalen Endabschnitt von dem länglichen, röhrenförmigen Teil (300) erstreckt, wobei der Finger (330) konfiguriert und angepasst ist, um mit einer chirurgischen Zielstelle zu kuppeln.

3. Das chirurgische Instrument gemäß Anspruch 1 oder 2, wobei die Zoomlinsenanordnung (150) eine optische Vergrößerungsleistung von mindestens 10 hat.

4. Das chirurgische Instrument gemäß einem vorangegangenen Anspruch, wobei das Lichtelement (120) ein kollimierter Emitter ist.

5. Das chirurgische Instrument gemäß einem vorangegangenen Anspruch, wobei die Muster (142) von der Maske (140) halbdurchsichtig sind.

6. Das chirurgische Instrument gemäß einem vorangegangenen Anspruch, wobei die Muster (142) von der Maske (140) eine Reihe von einheitlich beabstandeten konzentrischen Kreisen sind.

7. Das chirurgische Instrument gemäß einem vorangegangenen Anspruch, wobei das Handgriffteil (200) weiter ein LCD-Display (280) zum Betrachten der chirurgischen Zielstelle und der darauf projizierten Masken-(140)-Muster (142) beinhaltet.

8. Das chirurgische Instrument gemäß einem vorangegangenen Anspruch, wobei das Lichtelement (120) einen Lichtstrahl parallel zu einer longitudinalen Achse, die durch das chirurgische Instrument definiert wird, erzeugt.

9. Das chirurgische Instrument gemäß einem vorangegangenen Anspruch, wobei das Lichtelement (120) einen divergenten Lichtstrahl erzeugt.

10. Das chirurgische Instrument gemäß einem vorangegangenen Anspruch, wobei das chirurgische Instrument ein Endoskop ist.

**Revendications**

1. Instrument chirurgical pour utilisation en chirurgie invasive au minimum, comprenant :

un élément de manche (200) et
un élément tubulaire allongé (300) qui s'étend de manière distale de celui-ci,
un système de métrologie (100) monté sur l'élément tubulaire allongé (300) dans une partie d'extrémité distale de celui-ci,
**caractérisé en ce que** le système de métrologie (100) comprend :

un masque (140) comprenant des motifs (142) à projeter sur un site chirurgical d'intérêt pour en mesurer les dimensions ;
un ensemble de lentilles zoom (150) comprenant une pluralité d'éléments de lentille, au moins un élément de lentille de la pluralité d'éléments de lentille étant mobile ;
un élément lumineux (120) pour propager des faisceaux lumineux à travers le masque (140) et l'ensemble de lentilles zoom (150) pour projeter les motifs (142) du masque (140) sur le site chirurgical d'intérêt, et dans lequel l'élément de manche (200) comprend un cadran (220) couplé en service à l'ensemble de lentilles zoom (150) et dans lequel la rotation du cadran (220) entraîne le changement de grossissement des motifs (142) du masque (140) projetés sur le site chirurgical d'intérêt.

2. Instrument chirurgical selon la revendication 1, dans lequel l'élément tubulaire allongé (300) comprend un doigt (330) s'étendant de manière distale d'une partie d'extrémité distale de l'élément tubulaire allongé (300), le doigt (330) étant configuré et adapté pour s'engager sur un site chirurgical cible.

3. Instrument chirurgical selon la revendication 1 ou la revendication 2, dans lequel l'ensemble de lentilles zoom (150) a un pouvoir de grossissement optique d'au moins dix.

4. Instrument chirurgical selon l'une quelconque des revendications précédentes, dans lequel l'élément lumineux (120) est un émetteur collimaté.

5. Instrument chirurgical selon l'une quelconque des revendications précédentes, dans lequel les motifs (142) du masque (140) sont semi-transparents.

6. Instrument chirurgical selon l'une quelconque des revendications précédentes, dans lequel les motifs (142) du masque (140) sont une série de cercles concentriques uniformément espacés.

7. Instrument chirurgical selon l'une quelconque des revendications précédentes, dans lequel l'élément de manche (200) comprend en outre un affichage LCD (280) pour observer le site chirurgical cible et les motifs (142) du masque (140) qui y sont projetés.

8. Instrument chirurgical selon l'une quelconque des revendications précédentes, dans lequel l'élément lumineux (120) produit un faisceau lumineux parallèle à un axe longitudinal défini par l'instrument chirurgical.

9. Instrument chirurgical selon l'une quelconque des revendications précédentes, dans lequel l'élément lumineux (120) produit un faisceau lumineux divergent.

10. Instrument chirurgical selon l'une quelconque des revendications précédentes, dans lequel l'instrument chirurgical est un endoscope.

FIG. 1

FIG. 2

EP 2 630 915 B1

FIG. 4

FIG. 3

FIG. 5

## REFERENCES CITED IN THE DESCRIPTION

**Patent documents cited in the description**

- US 2010201796 A
- JP 2011185767 A
- US 2011279670 A
- US 2008024793 A